# EUROPEAN PATENT APPLICATION

(11) **EP 1 340 492 A1**
(43) Date of publication of application: **03.09.2003**
(21) Application number: 02004786.6
(22) Date of filing: 01.03.2002
(51) Int. Cl.: A61K 9/00, A61K 31/485

(54) **Aerosol formulations for pulmonary administration of medicaments having systemic effects**

(71) Applicant: CHIESI FARMACEUTICI S.p.A., 43100 Parma (IT)
(72) Inventor: Davies, Rebecca Jaine, 43100 Parma (IT); Ganderton, David, 43100 Parma (IT); Lewis, David Andrew, 43100 Parma (IT); Meakin, Brian John, 43100 Parma (IT); Brambilla, Gaetano, 43100 Parma (IT); Ferraris, Alessandra, 43100 Parma (IT)
(74) Representative: Minoja, Fabrizio, Dr.

(57) **Abstract**

The invention discloses an aerosol solution pharmaceutical composition comprising a medicament in a concentration of at least 0.01% w/v in a mixture comprising a hydrofluoroalkane propellant and one ore more co-solvents.

## Description

This invention relates to aerosol solution formulations comprising a medicament, a propellant, one or more co-solvents and optionally other additives commonly used in this kind of formulations.

### BACKGROUND OF THE INVENTION

Many pharmaceutically active compounds currently used in clinical practice and exhibiting problems of administration and/or absorption by the oral, parenteral or transdermal administration could take advantage from a pulmonary delivery, aimed at obtaining a systemic effect.

Pharmaceutically active compounds could be administered to the respiratory tract by using pressurised metered dose inhalers (pMDIs). PMDIs use a propellant to expel droplets containing the pharmaceutical product to the respiratory tract as an aerosol.

The formulation can be a solution or a suspension. Solution formulations, in comparison to suspensions, do not present problems of physical stability of the suspended particles and could therefore guarantee a higher dose uniformity and reproducibility.

As far as the propellant is concerned, hydrofluoroalkanes [(HFAs), known also as hydro-fluoro-carbons (HFCs)] would be mandatory propellants as chlorofluorocarbons (known also as Freons or CFCs), which were for many years the preferred propellants aerosols for pharmaceutical use, have been banned in view of their environmental impact.

In particular, 1,1,1,2-tetrafluoroethane (HFA 134a) and 1,1,1,2,3,3,3-heptafluoropropane (HFA 227) have been acknowledged as the best candidates for non-CFC propellants and a number of pharmaceutical aerosol formulations using such HFA propellants have been disclosed.

### DISCLOSURE OF THE INVENTION

The aim of providing solution formulations in a HFA propellant for aerosol delivery of medicaments is to give a prompt, systemically active dose of said medicaments *via* the respiratory tract.

Hereinafter, the term medicament is used to define any pharmaceutical active compound which could take advantage from a pulmonary delivery so as to produce a systemic therapeutic effect.

In order to provide therapeutically useful plasma levels, a therapeutic concentration of medicament and an efficient aerosol delivery should be achieved.

An important parameter for an efficient aerosol delivery producing a systemic therapeutic effect is the particle size distribution in the aerosol cloud. When the formulation is in the form of suspension, the particle size of the cloud is dominated by the particle size of the suspended drug, defined by the milling/micronization process.

When the formulation is in the form of solution, the volumetric contribution of suspended drug particles is absent and much finer liquid droplets clouds, largely defined by the drug concentration in the solution, are generated.

The size of the particles provided by the pMDI, is normally expressed as mass median aerodynamic diameter (MMAD). The particle size of choice for the treatment of bronchopulmonary diseases is usually of about 3 µm. The preferred diameter of the aerosol particles or droplets is comprised between 0.5 and 5 µm.

When the medicament is delivered to the lungs through an aerosol metered dose inhaler so as to produce a systemic effect, the particles should be small enough to be delivered to the lungs and to be absorbed into the bloodstream upon inhalation, i.e. of a size advantageously comprised between about 0.5 µm and 2.5 µm (MMAD of about 1-2 µm). Particles smaller than 0.5 µm are indeed not therapeutically useful as they are exhaled.

The aerosol solution formulations offer the advantage of being homogeneous with the active ingredient and with the excipients which are completely dissolved in the propellant vehicle or in the mixture thereof with suitable co-solvents such as ethanol. Solution formulations also obviate physical stability problems associated with suspension formulations,thus assuring reproducible dosage.

Furthermore, when a systemic effect is required, as in the case of the invention, aerosol solution formulations offer the advantage that much finer clouds, largely defined by the drug concentration in the solution, are generated and the finer clouds give more extensive deposition in the lung periphery.

When a medicament is slightly soluble in HFA propellants such as HFA 134a and HFA 227 or in their mixture,the use of a solvent, generally ethanol, is necessary.

When a medicament is very slightly soluble in the propellant, large amounts of ethanol are required. A large amount of ethanol, in turn, increases, proportionally to its concentration, the size of the aerosol droplets leaving the actuator orifice. The larger size droplets extensively deposit into the oropharyngeal tract, to the detriment of the drug dose fraction which penetrates into the lower airways (respirable fraction). A poorly respirable fraction is unlikely to give the medicament serum levels necessary to produce a therapeutic effect.

Moreover, an increased amount of ethanol in the formulation means also an increased amount of residual water. Whereas an amount of water comprised between 0.5-1% w/w may be in some cases useful to improve the solubility of the medicament in the propellant/co-solvent system, in other cases the presence of water could enhance the degradation of the medicament and could be detrimental to the physical stability of the formulation giving rise to a non-homogeneous system.

It would be advantageous to provide a formulation for pulmonary delivery to be used with pressurised metered dose inhalers, which is chemically and physically stable and capable of providing, on actuation, a suitable fine particle dose (FPD) and a fine respirable fraction (FPF), providing early therapeutical plasma levels of a medicament. The fine particle dose or respirable dose is the amount of active particles of size less than 4.7 µm and the fine particle fraction or respirable fraction is the ratio between the respirable dose and the dose delivered on actuation of the inhaler. The delivered respirable fraction should be at least 30%, preferably higher than 40%, even more preferably higher than 50% of the delivered dose.

It would also be highly advantageous to provide formulations whose delivered dose is highly reproducible after repeated administrations from the pMDI.

Since a high systemic exposure of the aerosol particles would, in this case, be of benefit, it would be even more advantageous to provide a formulation wherein the composition of the whole solvent system has been adjusted in order to allow the generation of aerosol particles which could then allow a deep lung penetration, at the same time minimizing the amount of very small particles (≤0.5 µm) which would be exhaled.

The invention provides a solution to said problems by means of solution formulations comprising at least 0.01% w/v of a medicament, an HFA propellant and optionally one or more co-solvents. Said solutions are chemically stable for an adequate time and capable of providing, on actuation, a respirable fraction giving rise to onset-hastened therapeutical plasma levels.

The preferred co-solvents are lower alkyl (C₁-C₄) alcohols, polyols, polyalkylene glycols and their combinations.

Ethanol is particularly preferred.

Other suitable co-solvents are (poly)alkoxy derivatives including polyalkoxy alcohols, in particular 2-(2-ethoxyethoxy) ethanol (available under the trademark Transcutol®).

Further (poly)alkoxy derivatives include polyoxyalkyl ethers and esters, such as polyoxyethylene ethers or esters. The preferred polyoxyethylene ethers and esters are polyoxyethylene alkyl ethers, polyoxyethylene sorbitan fatty acid esters and polyoxyethylene stearates.

As a cosolvent, a fatty acid alkyl ester can also be utilized. Ethyl oleate, isopropyl myristate and isopropyl palmitate are preferred.

According to a preferred embodiment, the invention provides a pharmaceutical composition comprising a medicament, a HFA propellant , ethanol in amount less than 10% w/w and a co-solvent with a higher polarity than ethanol in amount from 0.2% to 10% w/w, preferably from 0.5 to 10% w/w, more preferably from 0.5 to 5% w/w, even more preferably from 1 to 2% w/w. The addition of a co-solvent with a higher polarity than ethanol allows the reduction in the ethanol amount allowing the modulation of the particle size of the produced aerosol droplets.

Co-solvents with a higher polarity than ethanol can be preferably selected from lower alkyl (C₁-C₄) alcohols, a polyols or polyalkylene glycols.

The preferred polyols include propylene glycol and glycerol and the preferred polyalkylene glycol is polyethylene glycol.

According to an even more preferred embodiment, the invention, provides a pharmaceutical composition consisting essentially of a medicament, a HFA propellant, optionally ethanol in amounts comprised between 2 and 8% w/w, preferably between 5% and 8% w/w and optionally a co-solvent. The amount of the active ingredient is preferably comprised between 0.1 and 1.0% w/v.

Small amounts of ethanol and of a co-solvent are useful also when the medicament is fully soluble in the propellant.

It has been indeed found that, although the solvent is not needed to dissolve the medicament in the propellant, a small amount of ethanol (preferably comprised around 5-8% w/w, more preferably around 5% w/w),influencing the deposition characteristics, may make systemic delivery easier, since ethanol helps the reduction of the amount of very small particles (<0.5 µm) which would be exhaled due to the short residency time in the lung. Moreover, ethanol reduces the deposition of discharged material on the inhaler actuator orifice, so improving the dose reproducibility after repeated administrations by keeping *'clean'* the actuator orifice.

Due to this "cleaning" effect of ethanol, the use of surface active agents or "surfactants" as valve lubricants is not necessary.

In certain cases, however, the formulation may optionally contain small amounts of additional components such as surfactants or other additives, e.g. preservatives, buffers, antioxidants, sweeteners, taste masking agents.

The preferred organic surfactant is selected from oleyl alcohol, sorbitan trioleate, sorbitan mono-oleate, sorbitan monolaurate, polyoxyethylene (20) sorbitan monolaurate, polyoxyethylene (20) sorbitan mono-oleate, natural lecithin, oleyl polyoxyethylene (2) ether, stearyl polyoxyethylene (2) ether, lauryl polyoxyethylene (4) ether, block copolymers of oxyethylene and oxypropylene, oleic acid, synthetic lecithin, diethylene glycol dioleate, tetrahydrofurfuryl oleate, ethyl oleate, isopropyl myristate, glyceryl mono-oleate, glyceryl monostearate, glyceryl monoricinoleate, cetyl alcohol, stearyl alcohol, cetyl pyridinium chloride, olive oil, glyceryl monolaurate, corn oil, cotton seed oil or sunflower seed oil.

According to a further aspect, the invention provides a method of filling an aerosol inhaler with a composition of the invention, the method comprising:
(a) weighing the required quantity of active ingredient into the can or vial;
(b) adding the appropriate volume of ethanol and the other co-solvent , if required;
(c) crimping with valves and gassing;
(d) adding a propellant containing a hydrofluoroalkane (HFA).

Any medicament which can be administered by inhalation as aerosol, capable of being solubilized in an HFA/ethanol/co-solvent system and of being absorbed into the blood stream via the lung, may be used in the aerosol compositions of the invention. Examples of said medicaments are cyclooxygenase-, mast cell-, lipoxygenase- and proteolytic enzyme - inhibitors, arachidonic acid-, leukotriene-, thromboxane-, sodium/potassium channel-, neurokinin-, tachykinin-, bradykinin-, muscarine-, histamine-, phosphodiesterase- and selectin - antagonists, potassium channel blockers, anti-infective agents, antibiotics, pentamidine, cytostatics, fungistatics, free-radical scavengers, vitamins, hormones, immunostimulants, immunosuppressants, heparin, antidiabetics, analgesics, hypnotics and the like, for example:
- leukotriene antagonists such as iralukast, zafirlukast and pranlukast,
- a lipoxygenase inhibitor such as zileuton,
- sodium channel antagonists such as amiloride, potassium channel antagonists, bimakalim,
- arachidonic acid antagonists such as 2-benzoxazolamine,
- histamine receptor antagonists such as epinastine, azelastine, cinnarizine, cetrizine, mizolastine, mequitamium, chlorpheniramine, astemizole, terfenadine and fenoxfenadine,
- antimigrain agents such as ergot alkaloids methisergide, ergotamine, serotonin, sumatriptan, zolmitriptan, cyclandelate etc.,
- analgesics such as fentanyl, morphine, buprenorphine, opium, heroin, nalbuphine, pentazocine, oxycodone, tramadol, pethidine, tilidine, methadone, nefopam, dextropropoxyphene, piritramide, etc.,
- antiemetics such as bromopride, domperidone, metoclopramide, triethylperazine, trifluoropromazine, meclozine, chlorphenoxamine, dimenhydrinate etc.,
- antibiotics such as penicillins (e.g. azlocillin), cephalosporins (e.g. cefotiam or ceftriaxone), carbapenems, monobactams, aminoglycosides (e.g. streptomycin, neomycin, gentamycin, amikacin or tobramycin), quinolones (e.g. ciprofloxacin), macrolides (e.g. erythromycin), nitroimidazoles (e.g. tinidazol), lincosamide (e.g. clindamycin), glycopeptides (e.g. vancomycin), polypeptides (e.g. bacitracin), mupirocin etc.,
- vitamins and free-radical scavengers such as vitamin A, B, C, D or E, catalase, superoxide dismutase, reduced glutathione etc.,
- antidiabetics such as glibenclamide, glipizide, gliclazide, glimepiride, troglitazone etc.,
- hypnotics such as benzodiazepines, piperidonediones, antihistaminics etc.,
- neuroleptics, antidepressants and anticonvulsants such as benzodiazepines, phenothiazines, butyrophenones, sulpiride, hydantoins, barbiturates, succinimides, carbamazepine etc.,
- systemically active drugs such as, for example, isosorbide dinitrate, isosorbide mononitrate, apomorphine and cannabinoids,
- antiinflammatory agents,
- hormones such as androgens (e.g. testosteron), antioestrogens, calcitonin, parathyrin, somatotropin, oxytocin, prolactin, glucagon, erythropoietin, atriopeptin, melanotropin, thyrotropin, gonadotropin, vasopressin, insulin etc.,
- potency agent such as alprostadil,
- cytostatics such as nitrogen mustard derivatives (such as ifosphamide), N-Nitrosourea derivatives (e.g. lomustin), purine and pyrimidine bases antagonists (e.g. fluorouracil), platinum complexes (e.g. carboplatin), anthracyclines (e.g. doxorubicin), podophylline derivatives (e.g. podophyllotoxin).

Although the preferred medicaments of the invention are those usually not administered as a pulmonary aerosol, the aerosol solution formulations of the invention can be advantageously applied also to compounds already utilized in inhalation compositions, for instance a beta-mimetic such as salmeterol; a corticosteroid preferably selected from triamcinolone, ciclesonide, fluticasone and mometasone; an anticholinergic such as oxitropium bromide and thiotropium bromide; a mast cell inhibitor such as cromoglycic acid, nedocromil etc.

The high efficiency cloud generation allows the preparation of formulations containing a medicament with a reduced nominal dose and a larger percentage of clinically useful medicament deposition with respect to the reference composition (FPF of at least 30%, preferably higher than 40%, even more preferably more higher than 50% of the delivered dose) and with defined particle size targeting specific areas of the lungs.

Said medicaments can optionally be used in the form of esters, isomers, enantiomers or racemates thereof and, in the case of acids or bases, as such or in the form of their pharmaceutically acceptable salts.

Advantageously, the concentration of the active ingredient is at least 0.01% w/v, preferably at least 0.05% w/v, more preferably between 0.1% w/v and 1.0% w/v, even more preferably at least 1.0% w/v.

It is preferable that the formulation is suitable for delivering a therapeutic amount of the active ingredient in one or two actuations. Advantageously, the formulation will be suitable for delivering a therapeutic dose of at least 25 µg/dose, preferably between 50 and 500 µg/dose. By "therapeutic dose" it is meant the amount of active ingredient delivered by a single actuation of the inhaler able to produce a pharmacodynamic effect.

The formulations of the invention could be filled into cans suitable for delivering pharmaceutical aerosol formulations. Certain medicaments are subject to enhanced chemical degradation when stored in contact with the standard metal container usually made of aluminum. In these cases the formulations will be filled preferably into cans having part or all of the internal surfaces made of anodised aluminum, stainless steel or lined with an inert organic coating. Examples of preferred coatings are epoxy-phenol resins, perfluoroalkoxyalkane, perfluoroalkoxy alkylene, perfluoroalklenes such as polytetrafluoro ethylene, fluorinated-ethylene-propylene, polyether sulfone and a copolymer fluorinated-ethylene-propylene polyether sulfone. Other suitable coatings could be polyamide, polyimide, polyamideimide, polyphenylene sulfide or their combinations.

To further improve the stability, cans having a rolled-in rim and preferably a part or full rollover rim are used.

The formulation is actuated by a metering valve capable of delivering a volume of between 25 µl and 100 µl.

The choice of the metering valve and type of gasket will be made according the knowledge of the person skilled in the art. The gasket may comprise any suitable elastomeric material such as low density polyethylene, EPDM, chloroprene and TPE.

Suitable valves are commercially available from manufacturers well known in the aerosol industry, for example from Valois, France, Bespak plc, UK and 3M, Neotechnic Ltd, UK.

For reasons of chemical stability of the medicament in solution, it is preferred in some cases that the internal surfaces of metal valve components in contact with the formulation are coated with an inert material.

The currently used valve actuators with orifice diameter from 0.30 to 0.50 mm (and in particular 0.33, 0.42 and 0.45 mm) can be generally used with the aerosol formulations of the invention. When large amounts of ethanol are required to dissolve the medicament, so as to obtain aerosol clouds with an optimal respirable fraction, valve actuators provided with orifices with diameters comprised between 0.14-0.30 mm (and in particular 0.15, 0.22, 0.25 mm)are advantageously used.

These kinds of orifices can be prepared according to the EP application n° 01 130521.6, in the Applicant's name.

In some cases, in order to stabilize the medicament in solution, it would be necessary to provide aerosol solutions with a specific apparent pH, which can be determined by the skilled in the art according to WO 01/89480.

The hydrofluorocarbon propellant is preferably selected from HFA 134a, HFA 227 and mixtures thereof.

The co-solvent may include one or more solvents and in this case their ratio is a critical factor for an efficient aerosolization. The selection of said ratios may be anyhow made by the skilled in the art on the basis of the chemico-physical characteristics of the considered medicament.

The preferred co-solvents are usually alcohols such as ethanol, propanol, propylene glycol, polyethylene glycol and glycerol in an amount up to 25% w/w, preferably up to 15% w/w, more preferably up to 10% w/w, even more preferably up to 5%-8% w/w. The most preferred co-solvent is ethanol.

Advantageously, the droplets size is between about 0.5 µm and 2.5 µm, corresponding to a MMAD of about 1-2 µm.

### EXAMPLES

### Experimental part

### Preparation of HFA Solution pMDIs

The assembly of the pMDI cans was carried out using hand operated crimping and filling equipment. Formulations were prepared by accurately weighing the required quantity of drug into the can or vial. The appropriate volume of ethanol and the other co-solvent, if required, in the formulation was then added. The valve was crimped onto the vial/can and the assembled vial/can was ultra-sonicated for approximately 10 minutes. The HFA propellant was filled through the valve and the pMDI was ultra-sonicated for a further 10 minutes. In the case of formulations that contained drug and propellant only the pMDI was ultra-sonicated once, after the propellant had been added. Final compositions were calculated as percentage w/v for the active ingredient and as percentage w/w for the co-solvents.

### Solubility Studies

All solubility investigations were conducted in plastic coated glass pMDI vials fitted with continuous spray valves. Once produced the medicament-HFA solution pMDIs were stored in refrigerator at 4 °C (± 0.1 °C). The pMDI vials were removed periodically and the vials assessed visually with the aid of a polarized light unit for crystal growth.

### Cascade Impaction Studies

All impaction studies were conducted with formulations contained in cut edge anodised aluminium cans fitted with 50µl or 100µl valves. The studies were carried out using an Andersen Cascade Impactor (ACI) fitted with a USP XXII metal throat entry port.

The ACI was operated at a flow rate of 28.3 ± 2 I min ⁻¹. The HFA solution formulations were discharged into the ACI through actuators having an orifice diameter from 0.14 to 0.45 mm. Deposition of the drug on each ACI plate was determined by high pressure liquid chromatography (HPLC).

MMAD values and corresponding geometric standard deviation (GSD) were calculated from plots of the cumulative percentage undersize of drug collected on each ACI plate (probit scale), against the upper cut off diameter for each respective ACI plate (log10 scale).

The following parameters were determined: the metered dose, which is the sum of the dose delivered through the Andersen apparatus plus the active ingredient residue deposited on the device actuator; the cumulative amount of active particles deposited on the various ACI stages; the amount on the actuator; the amount in the adaptor and in the throat (adp/throat); the fine particle dose or respirable dose (FPD) which is the amount of particles deposited on stages 3 to filter of the ACI and corresponds to the amount of particles of size less than 4.7 µm; the fine particle fraction or respirable fraction which is the ratio between the respirable dose and the dose delivered ex-actuator.

### Example 1

### SOLUBILITY STUDIES OF DIISOBUTYROYL APOMORPHINE AND AEROSOL DELIVERY CHARACTERISTICS OF ITS CORRESPONDING pMDI FORMULATIONS

### Solubility studies

The solubility of diisobutyroyl apomorphine was investigated by producing pMDI formulations at various percentages of ethanol in HFA134a or in HFA 227.

The results showed that formulations containing up to 1% w/v diisobutyroyl apomorphine are soluble in HFA134a or HFA 227.

### Aerosol delivery characteristics studies

0.5% and 1% w/v (250µg or 500µg/50µl respectively) diisobutyroyl apomorphine HFA134a solution formulations containing 5% w/w ethanol and 0.1% w/w glycerol were produced. The cans were provided with actuators with an orifice diameter of 0.22mm.

Two ACI deposition determinations were performed with each formulation. Twenty shots were discharged into the ACI.

The diisobutyroyl apomorphine formulations prepared according to the invention presented a MMAD of about 2.0 µm, a fine particle fraction (FPF) of at least 70-75%, whereas the amount of active particles of sizes included in the range from 0.43 to 3.3 µm was of at least of 60%.

## Claims

1. An aerosol solution pharmaceutical composition comprising a medicament in a concentration of at least 0.01% w/v in a mixture comprising a hydrofluoroalkane propellant and one ore more co-solvents.

2. An aerosol solution pharmaceutical composition according to claim 1 wherein the aerosol liquid droplets delivered on actuation of the inhaler have a particle size comprised between 0.5 µm and 2.5 µm, with a mass median aerodynamic diameter of about 1-2 µm and wherein the fine particle fraction is at least 30% of the delivered dose.

3. A pharmaceutical formulation according to claim or 2, **characterised in that** the concentration of the medicament is at least 0.05% w/w.

4. A pharmaceutical formulation according to claims 1-3, wherein the propellant includes one or more HFAs selected from HFA 134a and HFA 227.

5. A pharmaceutical formulation according to claims 1-4, wherein the co-solvent is ethanol in an amount up to 10% w/w.

6. A pharmaceutical formulation according to claims 1-5, wherein the co-solvent is selected from ethanol, a lower alkyl (C₁-C₄) alcohol, a polyol or a polyalkylene glycol or mixtures thereof.

7. A pharmaceutical formulation according to claim 6 wherein polyols are selected from glycerol and propylene glycol and the polyalkylene glycol is polyethylene glycol.

8. A pharmaceutical formulation according to claims 1-5, wherein the co-solvent is a (poly)alkoxy derivative selected from polyalkoxy alcohols and polyoxyalkyl ethers and esters.

9. A pharmaceutical formulation according to claim 8, wherein the polyalkoxy alcohol is Transcutol®.

10. A pharmaceutical formulation according to claim 8, wherein the polyoxyalkyl ethers and esters are selected from polyoxyethylene alkyl ethers, polyoxyethylene sorbitan fatty acid esters and polyoxyethylene stearates.

11. A pharmaceutical formulation according to claims 1-5, wherein the co-solvent comprises a fatty acid alkyl ester selected from ethyl oleate, isopropyl myristate and isopropyl palmitate.

12. A pharmaceutical formulation according to any preceding claim, wherein the fine particle fraction is at least 40% of the delivered dose.

13. A pharmaceutical formulation according to any preceding claim, wherein the fine particle fraction is at least 50% of the delivered dose.

14. An aerosol inhaler containing a formulation as claimed in any of claims 1 to 13, wherein the valve actuator has an orifice diameter from 0.14 to 0.50 mm.

15. An aerosol inhaler containing a formulation as claimed in any of claims 1 to 13, wherein the valve actuator has an orifice diameter from 0.15 to 0.42 mm.

16. An aerosol inhaler containing a formulation as claimed in any of claims 1 to 13, wherein the valve actuator has an orifice diameter from 0.22 to 0.33 mm.
